# EUROPEAN PATENT APPLICATION

(11) **EP 3 009 362 A1**
(43) Date of publication of application: **20.04.2016**
(21) Application number: 14189314.9
(22) Date of filing: 17.10.2014
(51) Int. Cl.: B65B 55/08, A61L 2/10, B65B 55/10

(54) **A detecting device for detecting UV radiation**

(71) Applicant: Tetra Laval Holdings & Finance S.A., 1009 Pully (CH)
(72) Inventor: Ghirardello, Roberto, 41012 Carpi (IT); Gasparini, Enrico, 41126 Modena (IT)
(74) Representative: Tetra Pak - Patent Attorneys SE

(57) **Abstract**

A detecting device comprises a detecting sensor (52) for detecting UV radiation and a protecting screen (53) transparent to UV radiation and arranged for protecting a lens (54) of said detecting sensor (52), said detecting device further comprises an extension arrangement (55) provided with an elongated element (56) interposed between said detecting sensor (52) and said protecting screen (53) and defining a through channel (57), said channel (57) having a first end (58) covered by said protecting screen and a second end (59) facing said lens (54).

## Description

The present invention relates to a detecting device for detecting UV radiation. In particular, the detecting device may be used in a sterilizing unit of a packaging machine to detect the UV radiation used to sterilize a packaging material.

UV radiation has long been used in a wide range of applications. In the food industry, for example, it is commonly used for disinfecting or sterilizing packaging material, or for surface treating the food products themselves.

Various types of machines are known for packaging various types of pourable food products, such as fruit juice, wine, tomato sauce, pasteurized or long-storage (UHT) milk, etc.

In a known type of such machines packages are formed from a continuous tube of packaging material defined by a longitudinally sealed web.

The packaging material has a multilayer structure comprising a strong, stiff base layer, which may comprise fibrous material, such as paper, or material such as mineral-filled polypropylene. The base layer is covered on both sides with layers of heat-seal plastic material, such as polyethylene films; in the case of aseptic packages for long-storage products, such as UHT milk, the packaging material also comprises a layer of oxygen-barrier material, such as an aluminium foil or an ethyl vinyl alcohol (EVOH) foil, which is superimposed on a layer of heat-seal plastic material, and is in turn covered with another layer of heat-seal plastic material defining the inner face of the package eventually contacting the food product.

To produce aseptic packages, the web of packaging material is unwound off a reel and fed through a sterilizing unit, in which it is sterilized, for example, by immersion in a bath of liquid sterilizing agent, such as a concentrated solution of hydrogen peroxide and water.

Alternatively, or in addition to being treated with a liquid sterilizing agent, the web of packaging material may be treated by exposure to one or more sources of electromagnetic UV radiation.

UV sterilizing devices substantially comprise a UV radiation source housed in a casing and protected at the front by a screen made of material resistant and permeable to UV radiation.

Downstream from the sterilizing unit, the web of packaging material is dried, folded into a cylinder, and sealed longitudinally to form a continuous vertical tube of packaging material. The tube is filled continuously with the pourable food product, and is then fed to a forming and (transverse) sealing unit for producing the individual packages, and in which the tube is gripped between pairs of jaws and sealed transversely to form aseptic pillow packs.

The pillow packs are separated by cutting the sealed portions in between, and are then fed to a final folding station where they are folded mechanically into the final shape.

In one kind of the above-mentioned machines, the sterilizing units comprise a chamber positioned downstream of the bath of liquid sterilizing agent and through which the packaging material is advanced and exposed to the UV radiation.

The machines comprise a detecting device arranged to detect the amount UV radiation in the chamber, i.e. the amount of radiation that interacts with the packaging material to sterilize the packaging material.

The detecting device comprises a detecting sensor, i.e. a radiometer, fixed to a wall of the chamber. The detecting device also comprises a protecting screen made of a material resistant and permeable to the UV radiation emitted by the UV sterilizing device and arranged to protect a lens of the detecting sensor.

It may happen that the UV source of the UV sterilizing device is defective, or broken, and therefore, the amount of UV radiation transmitted to the packaging material is not enough to sterilize it. The detecting device - in case the detecting sensor detects a too low amount of UV radiation - stops the machine and generates an alarm.

The chamber is delimited by a first wall having a window that receives the UV sterilizing device, so that the UV sterilizing device can emit the UV radiation into the chamber. The chamber is also delimited by a second wall having a through hole extending from the outside of the chamber to the inside of the chamber. In particular, the hole has a first end zone facing the outer environment, i.e. the outside of the chamber, and a second end zone facing the inside of the chamber. The detecting sensor is fixed to the first wall at the first end zone of the hole so that the lens of the detecting sensor looks into the hole and the protecting screen is interposed between the first wall and the lens of the detecting sensor.

A drawback of the detecting device is that - especially at the start up of the machine, i.e. when the temperature inside the chamber is low - droplets of condensed vapor may accumulate on the protecting screen.

Due to the droplets of condensed vapor the detecting device may detect an amount of UV radiation that is lower than a working threshold and so generate an alarm that stops the packaging machine. The packaging machine, therefore, may be stopped due to a bad UV detection, even in case the UV sterilizing device works properly.

It is an object of the present invention to improve the detecting devices for detecting UV radiations, in particular the detecting devices used in sterilizing units of packaging machines.

Another object of the invention is to provide a detecting device in which bad reading, i.e. wrong detection, of a detecting sensor of the detecting device is reduced, or even eliminated.

Another object of the invention is to provide a detecting device in which the risk that droplets of condensed vapor accumulate on a protecting screen of a detecting sensor of the detecting device is reduced, or even eliminated.

According to the invention, there is provided a detecting device as claimed in claim 1.

A preferred, non-limiting embodiment of the present invention will be described by way of example with reference to the accompanying drawings, in which:
Figure 1 is a diagrammatic view of a machine for packaging pourable food products in packages made from a web of packaging material and having a sterilizing unit comprising a detecting device according to the invention;
Figure 2 is a larger-scale partly-exploded perspective view of a portion of the sterilizing unit of Figure 1;
Figure 3 is a perspective exploded view of the detecting unit according to the invention;
Figure 4 is a perspective view from above, with parts removed for clarity, of the sterilizing unit of Figure 1, which shows the detecting device according to the invention;
Figure 5 is a perspective partly-sectioned view from above showing a portion of the sterilizing unit of Figure 1 and the detecting device according to the invention.

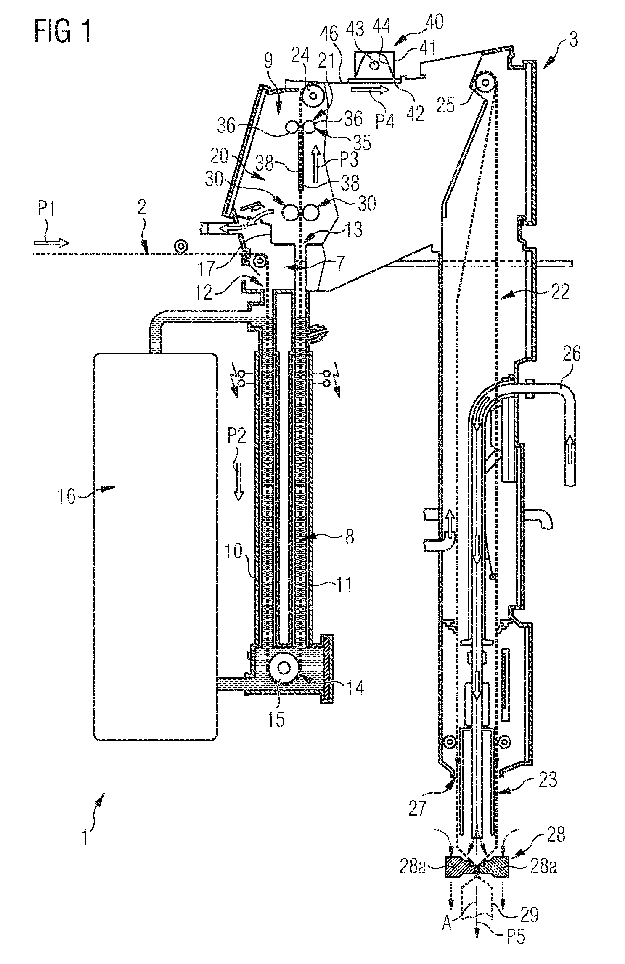

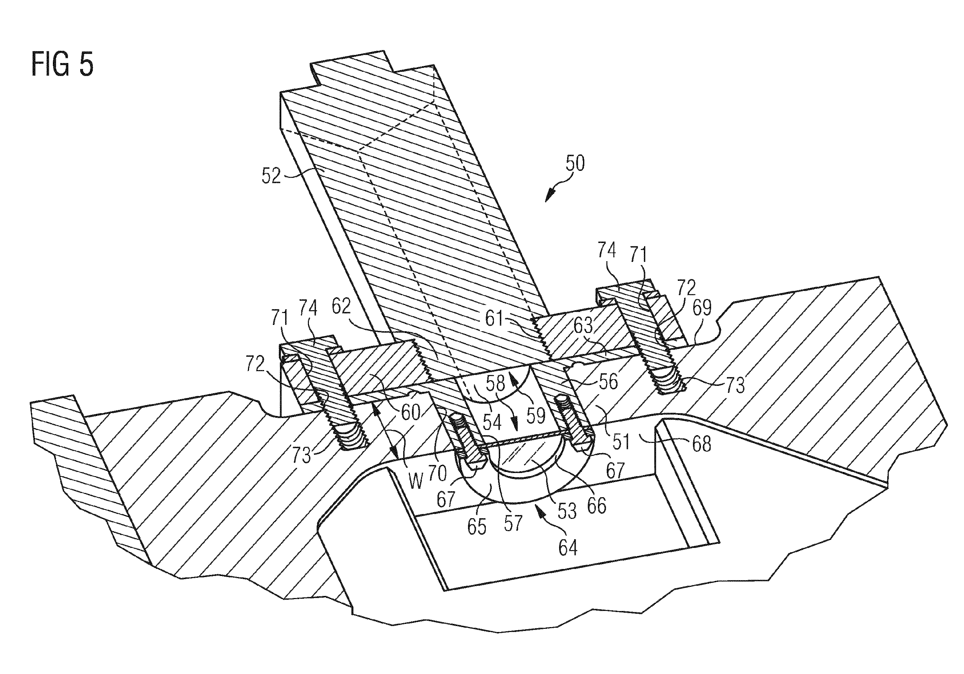

With reference to Figures 1 to 5, there is shown a packaging machine 1 for continuously producing aseptic sealed packages of a pourable food product from a web of packaging material 2 (hereafter referred to simply as "web 2").

The packaging machine 1 comprises a sterilizing unit 3, to which the web 2 is fed off a reel (not shown) along a path P1.

The sterilizing unit 3 comprises a transition chamber 7, into which the web 2 is first fed, a sterilizing bath 8 containing a liquid sterilizing agent, e.g. a 30% solution of hydrogen peroxide (H2O2) and water, through which the web 2 is advanced, and an aseptic chamber 9, in which the web 2 is dried, as explained in detail below.

The bath 8 is substantially defined by a U-shaped conduit filled, in use, with the sterilizing agent to a predetermined level. The U-shaped conduit is defined by an inlet branch 10 and by an outlet branch 11 having, respectively, a first top opening 12 and a second top opening 13, which define the inlet and outlet of the web 2 into and out of the bath 8, and communicate respectively with the transition chamber 7 and the aseptic chamber 9. The inlet branch 10 and the outlet branch 11 are connected at the bottom by a bottom portion 14 of the bath 8 housing a horizontal-axis guide roller 15, on which the web 2 is partially wound.

Inside the bath 8, the web 2 therefore describes a U-shaped path P2 of such a length as to keep the packaging material long enough inside the sterilizing agent.

The bath 8 is connected to a peroxide control circuit 16 - known and therefore not shown in detail - and is maintained, in use, at a controlled temperature, e.g. of around 70°C - 80°C.

The aseptic chamber 9 is separated from the transition chamber 7 by a partition 17 and has an inlet for web 2, coincident with the outlet (second top opening 13) of the bath 8.

The aseptic chamber 9 comprises a top portion 20, housing drying means 21 for removing residual sterilizing agent from the web 2, and a bottom portion 22 extending vertically and parallel to the bath 8, and in which the web 2 is folded longitudinally into a cylinder and sealed longitudinally to form a continuous cylindrical tube 23 having a vertical axis A.

The aseptic chamber 9 is maintained slightly above ambient pressure, so that any leakage through the seals occurs outwards as opposed to inwards of the chamber.

As visible in Figure 1, inside the top portion 20 of the aseptic chamber 9, the web 2 describes first a vertical path P3 extending on the prolongation of the vertical portion of path P2 in the outlet branch 11 and is then diverted by a first roller 24 and a second roller 25 onto a substantially horizontal path P4 and onto a vertical path P5 in the bottom portion 22, parallel to axis A of the tube 23.

In known manner and not described in detail, the tube 23, formed downstream from the second roller 25, is filled continuously with the food product for packaging by means of a fill conduit 26, and comes out downwards through a bottom opening 27 of the aseptic chamber 9, of which it substantially forms an extension.

The packaging machine 1 comprises a known transverse form-and-seal unit 28, not shown in detail, in which the tube 23 is gripped between pairs of jaws 28a, which seal the tube 23 transversely to form aseptic pillow packs 29 eventually formed by known cutting and folding operations into individual packages.

With reference to Figure 1, the drying means 21 comprise two idle squeeze rollers 30 having parallel horizontal axes, located close to the inlet of the aseptic chamber 9, on opposite sides of web 2, and at least one of which is covered with relatively soft material. The squeeze rollers 30 exert pressure on respective opposite faces of the web 2 to squeeze the drops of sterilizing agent out and back into the bath 8.

As shown in Figure 1, drying means 21 also comprise two so-called "air knives" 35 located on opposite sides of the web 2, downstream from the squeeze rollers 30 with reference to path P3 and therefore above the squeeze rollers 30.

Each air knife 35 comprises a nozzle 36 for directing a stream of air downwards (i.e. towards the bath 8) and on a respective face of the web 2 and, and a respective guide wall 38 that guides the respective stream of air, in use, in a direction substantially parallel to the web 2 but opposite to the travelling direction thereof.

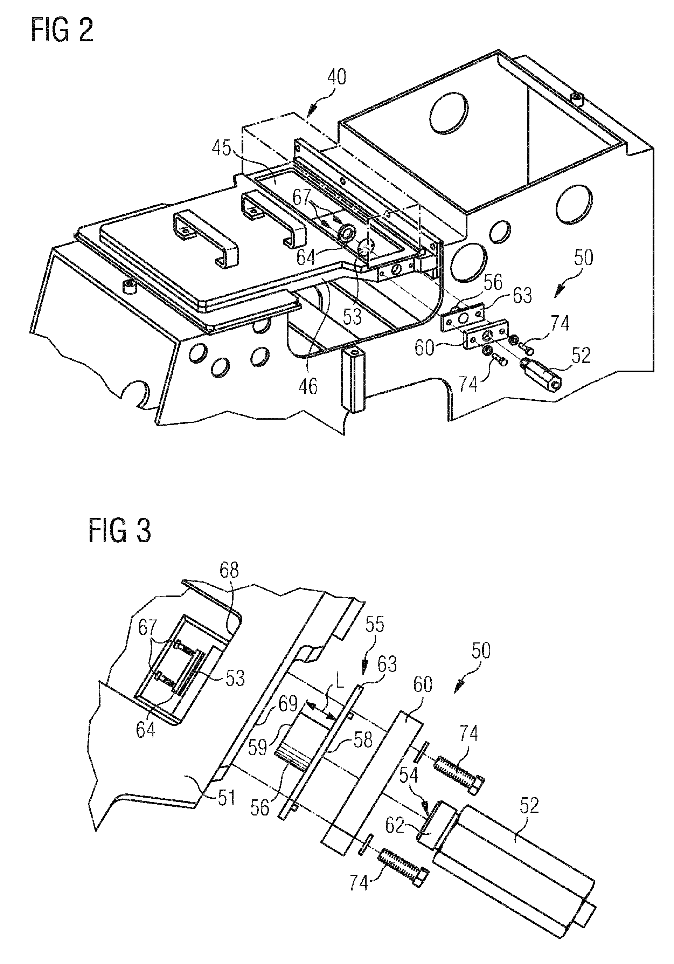

As shown in Figures 1 and 2, the sterilizing unit 3 further comprises a UV sterilizing device 40 positioned downstream of the sterilizing bath 8 and arranged to further sterilize the web 2.

The UV sterilizing device 40 is positioned along path P4.

The UV sterilizing device 40 comprises a casing 41 extending crosswise to the web 2 and open on the side of the web 2 so as to form a window 42.

The UV sterilizing device 40 also comprises a UV radiation source 43 housed longitudinally in the casing 41, which is provided with a reflecting element 44.

The UV radiation source 6 emits UV radiation, for example of a wavelength of 222 nm.

The window 42 faces an aperture 45 of an upper wall 46 of the aseptic chamber 9 so that the UV radiation is directed - through the window 42 and the aperture 45 - onto the web 2.

With reference to Figures 2 to 5, the sterilizing unit 3 further comprises a detecting device 50 for detecting the amount of UV radiation emitted by the UV sterilizing device 40 that interacts with the web 2. In particular, the detecting 50 device - in case it detects an amount of UV radiation that is below a certain threshold - generates an error signal and possibly stops the packaging machine 1, since the level of UV radiation may be not sufficient to assure a proper sterilization of the web 2. The low amount of UV radiation may be due to a defect, or a problem, in the UV sterilizing device.

The detecting device 50 is positioned in a lateral wall 51 of the aseptic chamber.

The detecting device 50 comprises a detecting sensor 52, for example a radiometer, for detecting UV radiation and a protecting screen 53 transparent to UV radiation for protecting a lens 54 of the detecting sensor 52.

In particular, the protecting screen 53 may be made of a polymer material. The polymer material may be of any type, provided it is transparent to UV radiation (T≥80%) and resistant to the UV radiation. The polymer material may, for example, be in the polyolefin group, such as PE or PP.

In one embodiment, the material is a fluorinated polymer, or a completely fluorinated polymer in the perfluoroalkoxy (PFA) class. In particular, the material is an MFA, e.g. the MFA available on the market under the trade name HYFLON® MFA.

The detecting device 50 comprises an extension arrangement 55 provided with an elongated element 56 interposed between the detecting sensor 52 and the protecting screen 53 and defining a through channel 57.

The channel 57 has a first end 58 covered by the protecting screen 53 and a second end 59 facing the lens 54.

The extension arrangement 55 also comprises a supporting body 60 provided with a through bore 61 that receives a portion 62 of the detecting sensor 52 supporting the lens 54, or comprising the lens 54.

The bore 61 is connected with the channel 57. In particular, the channel 57 and the bore 61 are substantially coaxial, the bore 61 having a diameter that is greater than a diameter of the channel 57.

In the embodiment shown, the elongated element 56 is distinct from the supporting body 60 and comprises a flange 63 fixed to the supporting body 60.

In addition, in the embodiment shown the protecting screen 53 is distinct from the elongated element 56 and is fixed to the elongated element 56 at the first end 58. In particular, the detecting device 50 comprises a fixing frame 64 for fixing the protecting screen 53 to the elongated element 56 and having a peripheral body 65 defining a window element 66. The fixing frame 64 and the protecting screen 53 are connected to the elongated element 56 by means of screws 67.

The lateral wall 51 has a first side 68 delimiting the inside of the aseptic chamber 9 and a second side 69, opposite to the first side 68, delimiting the outside of the aseptic chamber 9. A hole 70 extends through the lateral wall 51 from the first side 68 to the second side 69, i.e. between the outside of the aseptic chamber 9 and the inside of the aseptic chamber 9.

The elongated element 56 is received in the hole 70.

In this way, the first end 58 is close to the first wall 68 and the second end 59 is close to the second wall 59. In particular, the first end 58 is aligned with the first wall 68 and the second end 59 is aligned with the second wall 59. In addition, the flange 63 abuts against the second wall 69.

The elongated element 56 has an axial length L that is substantially equal to a width W of the lateral wall 51. In this way, the protecting screen 53 is arranged inside the aseptic chamber 9. In particular, the protecting screen 53 abuts against the first side 68.

The supporting body 60 has first holes 71 and the flange 63 has second holes 72. The lateral wall 51 has threaded holes 73.

The supporting body 60 and the elongated element 56 are fixed to the lateral wall 51 by means of screw elements 74 passing through the first holes 71 and the second holes 72 and mating with the threaded holes 73.

The supporting body 60 is made of heat insulating material, for example plastics, to protect the detecting sensor 52 from the heat generated by the hot air in the sterilizing unit 3.

During operation of the packaging machine 1, the web 2 is fed through the bath 8.

On entering the aseptic chamber 9 the web 2 is fed through the squeeze rollers 30, which squeeze the drops of residual sterilizing agent out and back into the bath 8.

At this point, the web 2 enters the air knives 35 and is swept by sterile-air streams ejected from the nozzles 36 and guided along the opposite faces of the web 2 by the guide walls 38.

Downstream of the the air knives 35, the web 2 is diverted by the roller 24 along path P4 and is further sterilized by the UV radiation generated by the UV sterilization device 40.

Subsequently the web is diverted by roller 25 along path P5 and is then folded into a cylinder and sealed longitudinally to form the tube 23, which is filled continuously with the pourable food product from the conduit 26, and is gripped and sealed transversely by jaws 28a to form a succession of pillow packs 29.

The advantages of the detecting device 50 according to the present invention will be clear from the foregoing description.

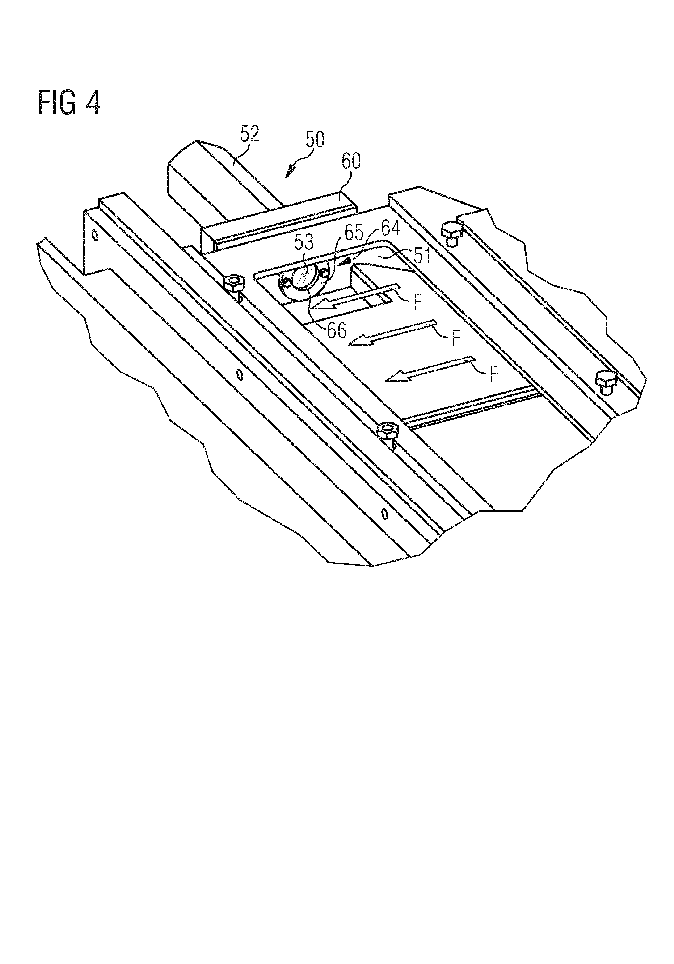

Owing to the extension arrangement 55, the protecting screen 53 is placed inside the aseptic chamber 9. In this way, the protecting screen 53 is continuously cleaned, or swept, by the stream of hot hair flowing in the aseptic chamber 9, as shown by the arrows F in Figure 4. In this way, droplets of condensed vapor are prevented from accumulating on the protecting screen 53. In any case, the droplets of condensed vapor - which may have formed on the protecting screen 53 - are immediately removed by the stream of hot air. In this way, the droplets of condensed water do not adversely affect the detecting capability of the detecting sensor 52. Therefore, no false alarm - due to a wrong detection caused by the droplets of condensed vapor - is generated by the detecting device 50.

This is not possible in the known UV sterilizing devices in which the protective screen is positioned between the lens and the wall of the aseptic chamber. In this case, in fact, the stream of hot air cannot effectively penetrate into the recess obtained in the wall of the aseptic chamber and, therefore, cannot prevent the droplets of condensed vapor from accumulating on the protecting screen.

Clearly, changes may be made to the detecting device as described and illustrated herein without, however, departing from the scope defined in the accompanying claims.

## Claims

1. A detecting device, comprising a detecting sensor (52) for detecting UV radiation and a protecting screen (53) transparent to UV radiation and arranged for protecting a lens (54) of said detecting sensor (52), **characterized in that** said detecting device further comprises an extension arrangement (55) provided with an elongated element (56) interposed between said detecting sensor (52) and said protecting screen (53) and defining a through channel (57), said channel (57) having a first end (58) covered by said protecting screen and a second end (59) facing said lens (54).

2. A detecting device according to claim 1, wherein said extension arrangement (55) comprises a supporting body (60) provided with a through bore (61) that receives a portion (62) of said detecting sensor (62) supporting said lens (54), said bore (61) being connected with said channel (57).

3. A detecting device according to claim 2, wherein said elongated element (56) is distinct from said supporting body (60) and comprises a flange (63) fixable to said supporting body (60).

4. A detecting device according to claim 2, or 3, wherein said channel (57) and said bore (61) are substantially coaxial, said bore (61) having a diameter that is greater than a diameter of said channel (57).

5. A detecting device according to any one of the preceding claims, wherein said protecting screen (53) is distinct from said elongated element and is fixed to said elongated element (56) at said first end (58).

6. A sterilizing unit for sterilizing a packaging material (2) adapted to be transformed in a plurality of sealed packages, said sterilizing unit (3) comprising a chamber (9) along which said packaging material (2) is advanced along a given path (PI, P2, P3, P4), a UV sterilizing device (40) having a UV radiation source (43) for directing a UV radiation on said packaging material (2) in said chamber (9) and a detecting device (50) according to any one of the preceding claims.

7. A sterilizing unit according to claim 6, wherein said chamber (9) is defined by a wall (51) having a first side (68) delimiting the inside of said chamber (9) and a second side (69), opposite to said first side (68), delimiting the outside of said chamber (9), and wherein a hole (70) extends through said wall (51) between said first side (58) and said second side (59), said elongated body (56) being received in said hole (70).

8. A sterilizing unit according to claim 7, wherein said protecting screen (53) is arranged inside said chamber (9).

9. A sterilizing unit according to claim 8, wherein said first end (58) is aligned with said first wall (68) and said second end (59) is aligned with said second wall (59).

10. A sterilizing unit according to claim 8, or 9, wherein said protecting screen (53) abuts against said first side (68).

11. A sterilization unit according to any one of claims 7 to 10, wherein said elongated element (56) has an axial length (L) that is substantially equal to a width (W) of said wall (51).
